# NEUE EUROPÄISCHE PATENTSCHRIFT

(11) **EP 0 991 469 B2**
(45) Veröffentlichungstag und Bekanntmachung des Hinweises auf die Entscheidung über den Einspruch: **23.08.2006**
(45) Hinweis auf die Patenterteilung: 27.02.2002
(21) Anmeldenummer: 98933596.3
(22) Anmeldetag: 05.06.1998
(51) Int. Cl.: B01J 37/00, C07D 301/12, C04B 38/08

(54) **FORMKÖRPER UND VERFAHREN ZU DESSEN HERSTELLUNG**
SHAPED BODY AND METHOD FOR THE PRODUCTION THEREOF
CORPS MOULE ET SON PROCEDE DE FABRICATION

(30) Priorität: 06.06.1997 DE 19723751
(43) Veröffentlichungstag der Anmeldung: 12.04.2000
(73) Patentinhaber: BASF Aktiengesellschaft, 67056 Ludwigshafen (DE)
(72) Erfinder: GROSCH, Georg, Heinrich, D-67098 Bad Dürkheim (DE); MÜLLER, Ulrich, D-67435 Neustadt (DE); WALCH, Andreas, D-69120 Heidelberg (DE); RIEBER, Norbert, D-68259 Mannheim (DE); HARDER, Wolfgang, D-69469 Weinheim (DE)
(74) Vertreter: Isenbruck, Günter
(86) Internationale Anmeldenummer: PCT/EP1998/003394
(87) Internationale Veröffentlichungsnummer: WO 1998/055229

(56) Entgegenhaltungen:
- EP-A- 0 072 390
- EP-A- 0 102 544
- EP-A- 0 197 645
- EP-A- 0 568 336
- EP-A- 0 639 404
- US-A- 4 162 285

## Beschreibung

Die vorliegende Erfindung betrifft einen einen Titansilikaliten enthaltenden Formkörper, ein Verfahren zu dessen Herstellung, sowie dessen Verwendung zur Umsetzung von organischen Verbindungen, insbesondere zur Epoxidation von organischen Verbindungen mit mindestens einer C-C-Doppelbindung. Der hierin beschriebene Formkörper weist hohe Abriebfestigkeit und ausgezeichnete mechanische Eigenschaften auf.

Abriebfeste Formkörper aus katalytisch aktiven Massen werden in vielen chemischen Verfahren eingesetzt, insbesondere bei Verfahren unter Verwendung eines Festbetts. Demgemäß existiert eine immense Fülle von Literatur zu diesem Thema. Über den Einsatz von Katalysatoren auf der Basis poröser oxidischer Materialien, wie z.B. Zeolithe, und speziell bezüglich der Verformung derartiger Materialien existiert bedeutend weniger Literatur.

In der Regel wird zur Herstellung von Festkörpem die katalytisch aktive Masse, d.h. das poröse oxidische Material mit einem Bindemittel, einer organischen viskositätssteigernden Verbindung und einer Flüssigkeit zum Anteigen der Masse versetzt und in einer Misch- oder Knetvorrichtung oder einem Extruder verdichtet. Anschließend wird die daraus resultierende plastische Masse verformt, insbesondere unter Verwendung einer Strangpresse oder eines Extruders, und die resultierenden Formkörper getrocknet und calciniert.

Als Bindemittel werden dabei eine Reihe von anorganischen Verbindungen benutzt.

So wird gemäß der US-A 5,430,000 Titandioxid oder Titandioxidhydrat als Bindemittel verwendet. Als weitere, im Stand der Technik genannte Bindemittel sind zu nennen:
Aluminiumoxidhydrat oder andere aluminiumhaltige Bindemittel (WO 94/29408);
Gemische aus Silizium- und Aluminiumverbindungen (WO 94/13584);
Siliziumverbindungen (EP-A 0 592 050);
Tonmineralien (JP-A 03 037 156);
Alkoxysilane (EP-B 0 102 544).

Als organische viskositätssteigernde Substanzen werden in der Regel hydrophile Polymere, wie z.B. Cellulose oder Polyacrylate, verwendet.

Ferner beschreibt die Anmelderin selbst in der DE-A 196 23 611.8 einen Oxidationskatalysator mit Zeolith-Struktur, der durch verfestigende Formgebungsprozesse geformt worden ist, sowie dessen Verwendung bei der Herstellung von Epoxiden aus Olefinen und Wasserstoffperoxid, sowie, in der DE-A 196 23 609.6 einen Oxidationskatalysator auf der Basis von Titan- oder Vanadiumsilicaliten mit Zeolith-Struktur, der ebenfalls durch verfestigende Formgebungsprozesse geformt worden ist und einen Gehalt von 0,01 bis 30 Gew.-% an einem oder mehreren Edelmetallen, wie darin definiert, aufweist.

In allen Druckschriften gemäß des oben zitierten Standes der Technik wird bei der Herstellung der dort beschriebenen Formkörper als Flüssigkeit zum Anteigen der Masse (Anteigungsmittel) Wasser verwendet.

Die EP-A 0 072 390 betrifft ein Verfahren zur Herstellung von Presslingen unter Verwendung von pyrogen hergestellten Oxiden. Derartige pyrogen hergestellte Oxide haben eine nicht-kristalline Struktur und zeichnen sich u. a. dadurch aus, daß sie keine Poren aufweisen.

Die US 4 162 285 betrifft die Herstellung von Keramik mit Bienenwaben-Struktur, wobei als Edukte Cordierit, Mullit, Aluminiumoxid, Zinkoxid, Siliciumnitrid oder eine Mischung aus zwei oder mehr davon eingesetzt wird.

Die EP-A 0 639 404 betrifft ein Verfahren zur Herstellung eines Katalysators, wobei die aktive Katalysatorkomponente zwingend Molybdän und Phosphor enthält.

Die oben beschriebenen auf einem porösen oxidischen Material basierenden Formkörper, wie z.B. Zeolithe und insbesondere Titansilicalite, besitzen jedoch einige Nachteile.

So besitzen viele der in der obigen Literatur beschriebenen Formkörper für eine Anwendung als Katalysator im Festbett nur eine unzureichende mechanische Festigkeit.

Dies fällt insbesondere dann ins Gewicht, wenn Nebenreaktionen bestimmter Bindemittel unerwünscht sind und aus diesem Grund ganze Klassen von Bindemitteln, die einem derartigen Formkörper eine ausreichende Festigkeit verleihen könnten, z.B. aufgrund anderer negativer Eigenschaften nicht verwendet werden können. Beispielsweise können bei der Herstellung von Titansilicalit, der als Katalysator für die Epoxidation von z.B. Propylen mit Wasserstoffperoxid verwendet wird, aluminiumhaltige Bindemittel nicht verwendet werden, da es aufgrund der durch den aluminiumhaltigen Bindemittel induzierten Acidität zu vermehrter Ringöffnung und Nebenproduktbildung kommt. Darüber hinaus können Titan-haltige Bindemittel zu hohen Zersetzungsraten des eingesetzten Wasserstoffperoxids führen, wenn diese Titan-haltigen Bindemittel zu nachweisbaren Titandioxidgehalten im Formkörper führen.

Ebenso unerwünscht ist es, Bindemittel zu verwenden, die einen Gehalt an Alkali- oder Erdalkalimetallen > 100 ppm besitzen. Durch Verwendung derartiger Bindemittel kann die katalytische Aktivität von z.B. Titansilicalit stark beeinträchtigt werden, da die katalytisch aktiven Ti-Zentren durch die Alkali- oder Erdalkaliionen inaktiviert werden.

Somit liegt der vorliegenden Erfindung die Aufgabe zugrunde, einen einen Titansilikaliten enthaltenden Formkörper, der eine ausreichende mechanische Stabilität aufweist, um als Katalysator in einem Festbett verwendet zu werden, bereitzustellen. Bei dessen Verwendung für katalytische Reaktionen sollte die aufgrund von Nebenreaktionen des zugesetzten Bindemittels auftretenden Aktivitäts- oder Selektivitätseinbußen verglichen mit den Katalysatoren gemäß des Standes der Technik vermieden werden. Ferner wird ein Verfahren zu dessen Herstellung bereitgestellt.

Überraschenderweise wurde gefunden, daß ein einen Titansilikaliten enthaltender Formkörper, der nahezu oder überhaupt keine Aktivitäts- und Selektivitätseinbußen bei seiner Verwendung als Katalysator aufweist, erhalten werden kann, sofern bei dessen Herstellung eine Mischung, enthaltend mindestens einen Alkohol und Wasser als Anteigungsmittel, und ein Tetraalkoxysilan oder ein Gemisch aus zwei oder mehr davon als Bindemittel verwendet werden.

Demgemäß betrifft die vorliegende Erfindung einen Titansilikaliten enthaltenden Formkörper, der erhältlich ist durch ein Verfahren, das die folgenden Stufen umfasst:
(I) Versetzen eines Gemischs, enthaltend einen Titansilikaliten und ein Tetraalkoxysilan oder ein Gemisch aus zwei oder mehr davon, mit einer Mischung, enthaltend mindestens einen Alkohol und Wasser,
(II) Kneten, Verformen, Trocknen und Calcinieren des gemäß Stufe (I) versetzten Gemischs,
wobei das Gemisch in Stufe (I) zusätzlich mit einem organischen hydrophilen Polymer oder einem Gemisch aus zwei oder mehr davon versetzt wird.
sowie ein Verfahren zur Herstellung eines mindestens einen Titansilikaliten enthaltenden Formkörpers, das die folgenden Stufen umfasst:
(I) Versetzen eines Gemischs, enthaltend einen Titansilikaliten und ein Tetraalkoxysilan oder ein Gemisch aus zwei oder mehr davon, mit einer Mischung, enthaltend mindestens einen Alkohol und Wasser.
(II) Kneten, Verformen, Trocknen und Calcinieren des gemäß Stufe (I) versetzten Gemischs,
wobei das Gemisch in Stufe (I) zusätzlich mit einem organischen hydrophilen Polymer oder einem Gemisch aus zwei oder mehr davon versetzt wird.

Die erfindungsgemäße Herstellung der oben beschriebenen Formkörper ausgehend von einem Titansilikaliten Pulverform beinhaltet die Bildung einer plastischen Masse, die mindestens den Titansilikaliten, ein Tetraalkoxysilan oder ein Gemisch aus zwei oder mehr davon als Bindemittel, eine Mischung enthaltend mindestens einen Alkohol und Wasser, ein organisches hydrophiles Polymers oder ein Gemisch aus zwei oder mehr davon als eine oder mehrere organische viskositätssteigernde Substanzen und weitere aus dem Stand der Technik bekannte Zusatzstoffe enthält.

Die durch inniges Vermischen, insbesondere Kneten der obigen Komponenten erhaltene plastische Masse wird vorzugsweise durch Strangpressen oder Extrudieren verformt und der erhaltene Formkörper wird nachfolgend getrocknet und abschließend calciniert.

Zeolithe sind bekanntermaßen kristalline Alumosilicate mit geordneten Kanal- und Käfigstrukturen, die Mikroporen aufweisen. Der Begriff "Mikroporen", wie er im Rahmen der vorliegenden Erfindung verwendet wird, entspricht der Definition in "Pure Appl. Chem." 45, S. 71 ff., insbesondere S. 79 (1976), und bezeichnet Poren mit einem Porendurchmesser von kleiner 2 nm. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W.M. Meier und D.H. Olson in "Atlas of Zeolithe Structure Types", Elsevier, 4. Auflage, London 1996.

Ferner existieren Zeolithe, die kein Aluminium enthalten und bei denen im Silicatgitter anstelle des Si(IV) teilweise Titan als Ti(IV) vorhanden ist. Die Titanzeolithe, insbesondere solche mit einer Kristallstruktur vom MFI-Typ, sowie Möglichkeiten zu ihrer Herstellung sind beschrieben, beispielsweise in der EP-A 0 311 983 oder der EP-A 0 405 978. Außer Silizium und Titan können solche Materialien auch zusätzliche Elemente wie Aluminium, Zirkonium, Zinn, Eisen, Kobalt, Nickel, Gallium, Bor oder geringe Mengen an Fluor enthalten.

In den beschriebenen Zeolithen kann das Titan desselben teilweise oder vollständig durch Vanadium, Zirkonium, Chrom, Niob oder Eisen ersetzt sein. Das molare Verhältnis von Titan und/oder Vanadium, Zirkonium, Chrom, Niob oder Eisen zur Summe aus Silizium und Titan und/oder Vanadium, Zirkonium, Chrom, Niob oder Eisen liegt in der Regel im Bereich von 0,01:1 bis 0,1:1.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Üblicherweise stellt man die genannten Titan-, Zirkonium-, Chrom-, Niob-, Eisen- und Vanadiumzeolithe dadurch her, daß man eine wäßrige Mischung aus einer SiO₂-Quelle, einer Titan-, Zirkonium-, Chrom-, Niob-, Eisen bzw. Vanadium-Quelle, wie z.B. Titandioxid bzw. einem entsprechenden Vanadiumoxid, Zirkoniumalkoholat, Chromoxid, Nioboxid oder Eisenoxid und einer stickstoffhaltigen organischen Base als Templat ("Schablonen-Verbindung"), wie z.B. Tetrapropylammoniumhydroxid, gegebenenfalls noch unter Hinzufügen von basischen Verbindungen, in einem Druckbehälter unter erhöhter Temperatur im Zeitraum mehrerer Stunden oder einiger Tage umsetzt, wobei ein kristallines Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan, bzw. das Zirkonium, Chrom, Niob, Eisen und/oder Vanadium zumindest teilweise innerhalb des Zeolithgerüsts in wechselndem Anteil mit 4-, 5- oder 6-facher Koordination vor. Zur Verbesserung der katalytischen Verhalens kann sich noch eine mehrmalige Waschbehandlung mit schwefelsaurer Wasserstoffperoxidlösung anschließen, worauf das Titan- bzw. Zirkonium-, Chrom-, Niob-, Eisen-, Vanadiumzeolith-Puiver erneut getrocknet und gebrannt werden muß; daran kann sich eine Behandlung mit Alkalimetallverbindungen anschließen, um den Zeolith von der H-Form in die Kation-Form zu überführen. Das so hergestellte Titan- bzw. Zirkonium-, Chrom-, Niob-, Eisen-, Vanadiumzeolith-Pulver wird dann, wie nachstehend beschrieben, zu einem Formkörper verarbeitet.

Titansilikalite sind Titan zeolithe mit Pentasil-Zeolith-Struktur, insbesondere die Typen mit röntgenographischer Zuordnung zur MFI-, MEL- oder MFI/MEL-Mischstruktur. Zeolithe dieses Typs sind beispielsweise in der oben angegebenen Literaturstelle von Meier und Olson beschrieben.

Auch bezüglich der Porenstruktur der erfindungsgemäßen Formkörper existieren keine besonderen Beschränkungen, d.h. der erfindungsgemäße Formkörper kann Mikroporen, Mesoporen, Makroporen, Mikro- und Mesoporen, Mikro- und Makroporen oder Mikro-, Meso- und Makroporen aufweisen, wobei die Definition der Begriffe "Mesoporen" und "Makroporen" ebenfalls derjenigen in oben erwähnter Literatur gemäß Pure Appl. Chem. entspricht und Poren mit einem Durchmesser von > 2 nm bis ca. 50 nm bzw. > ungefähr 50 nm bezeichnet.

Als Bindemittel eignen sich Oxide des Siliziums. Von besonderem Interesse als Bindemittel ist Siliziumdioxid, wobei das SiO₂ in Form von Tetraalkoxysilanen in den Formgebungsschritt eingebracht

Als Bindemittel wird ein Tetraalkoxysilan oder ein Gemisch aus zwei oder mehr davon als Bindemittel in Stufe (I) des erfindungsgemäßen Verfahrens zugesetzt.

Im Rahmen der vorliegenden Erfindung werden Tetraalkoxysilan als Bindemittel verwendet. Im einzelnen zu nennen sind dabei Tetramethoxysilan, Tetraethoxysilan, Tetrapropoxysilan und Tetrabutoxysilan, wobei Tetramethoxysilan und Tetraethoxysilan besonders bevorzugt sind.

Der erfindungsgemäße Formkörper enthält vorzugsweise bis zu ungefähr 80 Gew.-%, weiter bevorzugt ungefähr 1 bis ungefähr 50 Gew.-% und insbesondere ungefähr 3 bis ungefähr 30 Gew.-% Bindemittel, jeweils bezogen auf die Gesamtmasse des Formkörpers, wobei sich der Gehalt an Bindemittel aus der Menge des entstehenden Metalloxids ergibt.

Das verwendete Tetraalkoxysilan wird in einer solchen Menge eingesetzt, daß der daraus entstehende Metalloxid-Gehalt im Formkörper ungefähr 1 bis ungefähr 80 Gew.-%, vorzugsweise ungefähr 2 bis ungefähr 50 Gew.-% und insbesondere ungefähr 3 bis ungefähr 30 Gew.-%, jeweils bezogen auf die Gesamtmasse des Formkörpers liegt.

Wie sich aus obigem bereits ergibt, können selbstverständlich auch Gemische aus zwei oder mehr der oben genannten Bindemittel eingesetzt werden.

Essentiell für die vorliegende Erfindung ist es, daß bei der Herstellung des erfindungsgemäßen Formkörpers als Anteigungsmittel eine Mischung enthaltend mindestens einen Alkohol und Wassers verwendet wird. Dabei beträgt der Alkoholgehalt dieser Mischung im allgemeinen ungefähr 1 bis ungefähr 80 Gew.-%, vorzugsweise ungefähr 5 bis ungefähr 70 Gew.-% und insbesondere ungefähr 10 bis ungefähr 60 Gew.-%, jeweils bezogen auf das Gesamtgewicht der Mischung.

Vorzugsweise entspricht der verwendete Alkohol der Alkoholkomponente des als Bindemittel vorzugsweise verwendeten Metallsäureesters, wobei es jedoch auch nicht kritisch ist, einen anderen Alkohol zu verwenden.

Bezüglich der verwendbaren Alkohole bestehen keinerlei Beschränkungen, sofern sie wassermischbar sind. Es können demnach sowohl Monoalkohole mit 1 bis 4 C-Atomen und wassermischbare mehrwertige Alkohole verwendet werden. Insbesondere werden Methanol, Ethanol, Propanol sowie n-, iso-, tert.-Butanol, sowie Gemische aus zwei oder mehr davon verwendet.

Als organische viskositätssteigernde Substanz werden organische hydrophile Polymere, wie z.B. Cellulose, Stärke, Polyacrylate, Polymethacrylate, Polyvinylalkohol, Polyvinylpyrrolidon, Polyisobuten, Polytetrahydrofuran verwendet. Diese Substanzen fördern in erster Linie die Bildung einer plastischen Masse während des Knet-, Verformungs- und Trocknungsschritts durch Verbrücken der Primärpartikel und gewährleisten darüber hinaus die mechanische Stabilität des Formkörpers beim Verformen und Trocknen. Diese Substanzen werden beim Calcinieren wieder aus dem Formkörper entfernt.

Als weitere Zusatzstoffe können Amine oder aminartige Verbindungen, wie z.B. Tetraalkylammoniumverbindungen oder Aminoalkohole, sowie carbonathaltige Substanzen, wie z.B. Calciumcarbonat, zugesetzt werden. Derartige weitere Zusatzstoffe sind in EP-A 0 389 041, EP-A 0 200 260 und in WO 95/19222 beschrieben, die diesbezüglich vollumfänglich in den Kontext der vorliegenden Anmeldung durch Bezugnahme einbezogen werden.

Statt basischer Zusatzstoffe ist es auch möglich saure Zusatzstoffe zu verwenden. Diese können unter anderem eine schnellere Reaktion des Metallsäureesters mit dem porösen oxidischen Material bewirken. Bevorzugt sind organische saure Verbindungen, die sich nach dem Verformungsschritt durch Calcinieren herausbrennen lassen. Besonders bevorzugt sind Carbonsäuren. Selbstverständlich können auch Gemische aus zwei oder mehr der oben genannten Zusatzstoffe eingebaut werden.

Die Zugabereihenfolge der Bestandteile der den Titansilikaliten enthaltenden Masse ist nicht kritisch. Es ist sowohl möglich, zuerst das Bindemittel zuzugeben, anschließend die organische viskositätssteigernde Substanz, ggf. den Zusatzstoff und zum Schluß die Mischung enthaltend mindestens einen Alkohol und Wassers, als auch die Reihenfolge bezüglich des Bindemittels, der organischen viskositätssteigernden Substanz und der Zusatzstoffe zu vertauschen.

Nach der Zugabe des Bindemittels zum pulverförmigen Titansilikaliten dem gegebenenfalls die organische viskositätssteigernde Substanz bereits zugegeben worden ist, wird die in der Regel noch pulverförmige Masse 10 bis 180 Minuten im Kneter oder Extruderhomogenisiert. Dabei wird in der Regel bei Temperaturen im Bereich von ungefähr 10 °C bis zum Siedepunkt des Anteigungsmittel und Normaldruck oder leichtem überathmosphärischem Druck gearbeitet. Danach erfolgt die Zugabe der restlichen Bestandteile, und das so erhaltene Gemisch wird solange geknetet, bis eine verstrangbare oder extrudierfähige, plastische Masse entstanden ist.

Prinzipiell können für die Knetung und die Verformung alle herkömmlichen Knet- und Verformungsvorrichtungen bzw. Verfahren, wie sie zahlreich aus dem Stand der Technik bekannt sind und für die Herstellung von z.B. Katalysator-Formkörpern allgemein verwendet werden.

Wie bereits angedeutet, sind jedoch Verfahren bevorzugt, bei denen die Verformung durch Extrusion in üblichen Extrudern, beispielsweise zu Strängen mit einem Durchmesser von üblicherweise ungefähr 1 bis ungefähr 10 mm, insbesondere ungefähr 2 bis ungefähr 5 mm, erfolgt. Derartige Extrusionsvorrichtungen werden beispielsweise in Ullmanns Enzyklopädie der Technischen Chemie, 4. Auflage, Bd. 2, S. 295 ff., 1972 beschrieben. Neben der Verwendung eines Extruders wird ebenfalls vorzugsweise eine Strangpresse zur Verformung verwendet.

Nach Beendigung des Strangpressens oder Extrudierens werden die erhaltenen Formkörper bei im allgemeinen ungefähr 30 °C bis 140 °C (1 bis 20 h, Normaldruck) getrocknet und bei ungefähr 400 °C bis ungefähr 800 °C (3 bis 10 h, Normaldruck) calciniert.

Selbstverständlich können die erhaltenen Stränge bzw. Extrudate zerkleinert werden. Sie werden dabei vorzugsweise zu einem Granulat oder Splitt mit einem Partikeldurchmesservon 0,1 bis 5 mm, insbesondere 0,5 bis 2 mm zerkleinert.

Dieses Granulat oder dieser Splitt und auch auf anderem Wege erzeugte Formkörper enthalten praktisch keine feinkörnigeren Anteile als solche mit ungefähr 0,1 mm Mindestpartikeldurchmesser.

Die erfindungsgemäßen bzw nach dem erfindungsgemäßen Verfahren hergestellten, einen Titansilikaliten enthaltenden Formkörper besitzen . - verglichen mit entsprechenden Formkörpern des Standes der Technik - eine verbesserte mechanische Stabilität bei gleichzeitigem Erhalt der Aktivität und Selektivität.

Die erfindungsgemäßen bzw. erfindungsgemäß hergestellten Formkörper können zur katalytischen Umwandlung organischer Moleküle eingesetzt werden. Umsetzungen dieser Art sind beispielsweise Oxidationen, die Epoxidation von Olefinen wie z.B. die Herstellung von Propylenoxid aus Propylen und H₂O₂, die Hydroxylierung von Aromaten, wie z.B. Hydrochinon aus Phenol und H₂O₂, die Umwandlung von Alkanen zu Alkoholen, Aldehyden und Säuren, Isomerisierungsreaktionen, wie z.B. die Umwandlung von Epoxiden zu Aldehyden, sowie weitere in der Literatur mit derartigen Formkörpern, insbesondere Zeolith-Katalysatoren beschriebenen Umsetzungen, wie sie beispielsweise in W. Hölderich, "Zeolites: Catalysts for the Synthesis of Organic Compounds", Elsevier, Stud. Surf. Sci. Catal., 49, Amsterdam (1989), S. 69 bis 93, und insbesondere für mögliche Oxidationsreaktionen von B. Notari in Stud. Surf. Sci. Catal., 37 (1987), S. 413 bis 425, beschrieben sind.

Dabei eignet sich der vorstehend ausführlich diskutierte Titansilikalit insbesondere für die Epoxidation von Olefinen, vorzugsweise solchen mit 2 bis 8 C-Atomen, weiter bevorzugt Ethylen, Propylen oder Buten, und insbesondere Propen zu den entsprechenden Olefinoxiden. Demgemäß betrifft die vorliegende Erfindung insbesondere die Verwendung des hierin beschriebenen Formkörpers zur Herstellung von Propylenoxid ausgehend von Propylen und Wasserstoffperoxid.

Darüber hinaus betrifft die vorliegende Erfindung in ihrer allgemeinsten Ausgestaltungsform die Verwendung einer Mischung enthaltend mindestens einen Alkohol und Wasser als Anteigungsmittel, in Kombination mit einem Tetraalkoxysilan als Bindemittel zur Herstellung verformbarer Gemische, die einen Titansilikaliten enthalten.

### BEISPIELE

### Beispiel 1

In einem Vierhalskolben (4 l Inhalt) wurden 910 g Tetraethylorthosilicat vorgelegt und aus einem Tropftrichter innerhalb von 30 min mit 15 g Tetraisopropylorthotitanat unter Rühren (250 U/min, Blattrührer) versetzt. Es bildete sich eine farblose, klare Mischung. Anschließend versetzte man mit 1600 g einer 20 gew.-%igen Tetrapropylammoniumhydroxid-Lösung (Alkaligehalt < 10 ppm) und rührte noch eine Stunde nach. Bei 90 °C bis 100 °C wurde das aus der Hydrolyse gebildete Alkoholgemisch (ca. 900 g) abdestilliert. Man füllte mit 3 I Wasser auf und gab das mittlerweile leicht opaque Sol in einen 5 l fassenden Rührautoklaven aus Edelstahl.

Mit einer Heizrate von 3 °C/min wurde der verschlossene Autoklav (Ankerrührer, 200 U/min) auf eine Reaktionstemperatur von 175 °C gebracht. Nach 92 Stunden war die Reaktion beendet. Das erkaltete Reaktionsgemisch (weiße Suspension) wurde abzentrifugiert und mehrfach mit Wasser neutral gewaschen. Der erhaltene Feststoff wurde bei 110 °C innerhalb von 24 Stunden getrocknet (Auswaage: 298 g). Anschließend wurde unter Luft bei 550 °C in 5 Stunden das im Zeolithen verbliebene Templat abgebrannt. (Calcinierungsverlust: 14 Gew.-%).

Das reinweiße Produkt hatte nach naßchemischer Analyse einen Ti-Gehält von 1,5 Gew.-% und einen Gehalt an Restalkali unterhalb 100 ppm. Die Ausbeute auf eingesetztes SiO₂ betrug 97 %. Die Kristallite hatten eine Größe von 0,05 bis 0,25 µm und das Produkt zeigte im IR eine typische Bande bei ca. 960 cm⁻¹.

### Beispiel 2

120 g Titansilicalit-Pulver, synthetisiert gemäß Beispiel 1, wurden mit 48 g Tetramethoxysilan 2 h lang im Kneter vermischt. Anschließend wurden 6 g Walocel (Methylcellulose) zugegeben. Zum Anteigen gab man nun 77 ml einer Wasser-Methanol-Mischung mit einem Methanolgehalt von 25 Gew.-% zu. Die erhaltene Masse wurde weitere 2 h im Kneter verdichtet und dann in einer Strangpresse zu 2 mm-Strängen verformt. Die erhaltenen Stränge wurden bei 120 °C 16 h lang getrocknet und dann bei 500 °C 5 h lang calciniert. Die so erhaltenen Formkörper wurden auf ihre Seitendruckfestigkeit geprüft. Die Seitendruckfestigkeit betrug 4,11 kg.10 g der so erhaltenen Formkörper wurden zu Splitt (Partikelgröße 1 - 2 mm) verarbeitet und als Katalysator A in der Epoxidation von Propen mit Wasserstoffperoxid verwendet.

### Vergleichsbeispiel 1

120 g Titansilicalit-Pulver, synthetisiert gemäß Beispiel 1, wurden mit 48 g Tetramethoxysilan 2 h lang im Knetervermischt. Anschließend wurden 6 g Walocel (Methylcellulose) zugegeben. Zum Anteigen gab man nun 80 ml Wasser zu. Die erhaltene Masse wurde weitere 2 h im Kneter verdichtet und dann in einer Strangpresse zu 2 mm-Strängen verformt. Die erhaltenen Stränge wurden bei 120 °C 16 h lang getrocknet und dann bei 500 °C 5 h lang calciniert. Die so erhaltenen Formkörperwurden auf ihre Seitendruckfestigkeit geprüft. Die Seitendruckfestigkeit betrug 3,59 kg. 10 g der so erhaltenen Formkörper wurden zu Splitt (Partikelgröße 1 - 2 mm) verarbeitet und als Katalysator B in der Epoxidation von Propen mit Wasserstoffperoxid verwendet.

### Beispiel 3

120 g Titansilicalit-Pulver, synthetisiert gemäß Beispiel 1, wurden trocken mit 6 g Walocel (Methylcellulose) gemischt und mit 48 g Tetraethoxysilan 30 min im Kneter vermischt. Zum Anteigen gab man nun 75 ml einer Wasser-Ethanol-Mischung mit einem Ethanolgehalt von 50 Gew-% zu. Die so erhaltene Masse wurde 1 h lang im Kneter verdichtet und dann in einer Strangpresse zu 2 mm-Strängen verformt. Die so erhaltenen Stränge wurden bei 120 °C 16 h lang getrocknet und dann bei 500 °C 5 h lang calciniert. Die so erhaltenen Formkörper wurden auf ihre Seitendruckfestigkeit geprüft. Die Seitendruckfestigkeit betrug 3,08 kg. 10 g der so erhaltenen Formkörper wurden zu Splitt (Partikelgröße 1 - 2 mm) verarbeitet und als Katalysator C in der Epoxidation von Propen mit Wasserstoffperoxid verwendet.

### Vergleichsbeispiel 2

120 g Titansilicalit-Pulver, synthetisiert gemäß Beispiel 1, wurden mit 48 g Tetraethoxysilan 2 h im Kneter vermischt. Anschließend wurden 6 g Walocel (Methylcellulose) zugegeben. Zum Anteigen gab man nun 79 ml Wasser zu. Die so erhaltene Masse wurde 1 h lang im Kneter verdichtet und dann in einer Strangpresse zu 2 mm-Strängen verformt. Die erhaltenen Stränge wurden bei 120 °C 16 h lang getrocknet und dann bei 500 °C 5 h lang calciniert. Die so erhaltenen Formkörper wurden auf ihre Seitendruckfestigkeit geprüft. Die Seitendruckfestigkeit betrug 1,92 kg. 10 g der so erhaltenen Formkörper wurde zu Splitt (Partikelgröße 1 - 2 mm) verarbeitet und als Katalysator D in der Epoxidation von Propen mit Wasserstoffperoxid verwendet.

### Vergleichsbeispiel 3

120 g Titansilicalit-Pulver, synthetisiert gemäß Beispiel 1, wurden mit 6 g Walocel (Methylcellulose), 30 g Kieselsol (Ludox AS-40) und 85 ml Wasser 2 h im Kneter verdichtet. Die so erhaltene Masse wurde dann in einer Strangpresse zu
2 mm-Strängen verformt. Die erhaltenen Stränge wurden bei 120 °C 16 h lang getrocknet und dann bei 500 °C 5 h lang calciniert. Die so erhaltenen Formkörper wurden auf ihre Seitendruckfestigkeit geprüft. Die Seitendruckfestigkeit betrug 0,89 kg. 10 g der so erhaltenen Formkörper wurden zu Splitt (Partikelgröße 1 - 2 mm) verarbeitet und als Katalysator E in der Epoxidation von Propen mit Wasserstoffperoxid verwendet.

### Beispiele 4 bis 8

In einen Stahlautoklaven mit Korbeinsatz und Begasungsrührerwurden jeweils soviel Gramm an Katalysator A bis E eingebaut, daß die Masse an eingebautem Titansilicalit 0,5 g betrug. Der Autoklav wurde mit 100 g Methanol befüllt, verschlossen und auf seine Dichtigkeit überprüft. Anschließend wurde er auf 40 °C temperiert und 11 g flüssiges Propen in den Autoklaven dosiert. Nun wurden mittels einer HPLC-Pumpe 9,0 g einer wäßrigen Wasserstoffperoxidlösung (Gehalt an Wasserstoffperoxid in der Lösung 30 Gew.-%) in den Autoklaven gepumpt und die Wasserstoffperoxidreste in den Zuleitungen anschließend mit 16 ml Methanol in den Autoklaven gespült. Der Anfangsgehalt der Reaktionslösung an Wasserstoffperoxid betrug 2,5 Gew- %. Nach 2 h Reaktionszeit wurde der Autoklav abgekühlt und entspannt. Der flüssige Austrag wurde cerimetrisch auf Wasserstoffperoxid untersucht. Die Analyse und die Bestimmung des Gehalts an Propylenoxid erfolgte gaschromatographisch.

| Katalysator | Gehalt an Propylenoxid (Gew.-%) | Restgehalt an Wasserstoffperoxid (Gew.-%) |
|---|---|---|
| A | 1,42 | 0,99 |
| B (Vergleich) | 1,19 | 1,12 |
| C | 1,28 | 1,10 |
| D (Vergleich) | 1,15 | 1,20 |
| E (Vergleich) | 1,49 | 0,98 |

Aus den Beispielen ist ersichtlich, daß durch die erfindungsgemäße Verwendung von Tetraalkoxysilanen als Bindemittel und Wasser-Alkoholmischungen als Anteigungsmittel eine erhöhte Seitendruckfestigkeit bei gleichzeitigem Erhalt der Selektivität und Aktivität erhalten werden kann.

## Patentansprüche

1. Ein einen Titansilikaliten enthaltender Formkörper, der erhältlich ist durch ein Verfahren, das die folgenden Stufen umfasst:
(I) Versetzen eines Gemischs, enthaltend einen Titansilikaliten und ein Tetraalkoxysilan oder ein Gemisch aus zwei oder mehr davon, mit einer Mischung, enthaltend mindestens einen Alkohol und Wasser,
(II) Kneten, Verformen, Trocknen und Calcinieren des gemäß Stufe (I) versetzten Gemischs,
wobei das Gemisch in Stufe (I) zusätzlich mit einem organischen hydrophilen Polymer oder einem Gemisch aus zwei oder mehr davon versetzt wird.

2. Verfahren zur Herstellung eines mindestens einen Titansilikaliten enthaltenden Formkörpers, das die folgenden Stufen umfasst:
(I) Versetzen eines Gemischs, enthaltend einen Titansilikaliten und ein Tetraalkoxysilan oder ein Gemisch aus zwei oder mehr davon, mit einer Mischung, enthaltend mindestens einen Alkohol und Wasser.
(II) Kneten, Verformen, Trocknen und Calcinieren des gemäß Stufe (I) versetzten Gemischs,
wobei das Gemisch in Stufe (I) zusätzlich mit einem organischen hydrophilen Polymer oder einem Gemisch aus zwei oder mehr davon versetzt wird.

3. Verfahren nach Anspruch 2, wobei der Alkohol in der Mischung, enthaltend mindestens einen Alkohol und Wasser, mit dem Alkohol im Tetraalkoxysilan übereinstimmt.

4. Verfahren nach Anspruch 2 oder 3, wobei das in der Stufe (I) erhaltene Gemisch durch Strangpressen oder Extrudieren verformt wird.

5. Verfahren nach einem der Ansprüche 2 bis 4, wobei der einen Titansilikaliten enthaltende Formkörper Mikroporen, Mesoporen, Makroporen, Mikro- und Mesoporen, Mikro- und Makroporen oder Mikro-, Meso- und Makroporen aufweist.

6. Verwendung eines Formkörpers gemäß Anspruch 1 oder eines Formkörpers, hergestellt durch ein Verfahren gemäß einem der Ansprüche 2 bis 5, oder eines Gemischs aus zwei oder mehr davon zur Epoxidation von organischen Verbindungen mit mindestens einer C-C-Doppelbindung, zur Hydroxylierung von aromatischen organischen Verbindungen oder zur Umwandlung von Alkanen zu Alkoholen, Ketonen, Aldehyden und Säuren.

7. Verwendung eines Formkörpers gemäß Anspruch 1 oder eines Formkörpers, hergestellt durch ein Verfahren gemäß einem der Ansprüche 2 bis 5, zur Epoxidation eines Olefins, vorzugsweise zur Herstellung von Propylenoxid ausgehend von Propylen und Wasserstoffperoxid.

8. Verwendung einer Mischung, enthaltend mindestens einen Alkohol und Wasser, als Anteigungsmittel in Kombination mit einem Tetraalkoxysilan als Bindemittel zur Herstellung eines verformbaren Gemischs, das einen Titansilikaliten enthält.

## Claims

1. A molding containing a titanium silicalite and obtainable by a process which comprises the following stages:
(I) addition of a mixture containing at least one alcohol and water to a mixture containing a titanium silicalite and a tetraalkoxysilane or a mixture of two or more thereof, and
(II) kneading, molding, drying and calcination of the mixture according to stage (I) after addition,
wherein an organic hydrophilic polymer or a mixture of two or more thereof is additionally added to the mixture in stage (I).

2. A process for the production of a molding containing at least one titanium silicalite, which comprises the following stages:
(I) addition of a mixture containing at least one alcohol and water to a mixture containing a titanium silicalite and a tetraalkoxysilane or a mixture of two or more thereof, and
(II) kneading, molding, drying and calcination of the mixture according to stage (I) after addition,
wherein an organic hydrophilic polymer or a mixture of two or more thereof is additionally added to the mixture in stage (I).

3. A process as claimed in claim 2, wherein the alcohol in the mixture containing at least one alcohol and water corresponds to the alcohol in the tetraalkoxysilane.

4. A process as claimed in claim 2 or 3, wherein the mixture obtained in stage (I) is molded by extrusion pressing or extruding.

5. A process as claimed in any of claims 2 to 4, wherein the molding containing a titanium silicalite has micropores, mesopores, macropores, micro- and mesopores, micro- and macropores or micro-, meso- and macropores.

6. The use of the molding as claimed in claim 1 or of a molding produced by a process as claimed in any of claims 2 to 5 or of a mixture of two or more thereof for the epoxidation of organic compounds having at least one C-C double bond, for the hydroxylation of aromatic organic compounds, or for the conversion of alkanes to alcohols, ketones, aldehydes and acids.

7. The use of the molding as claimed in claim 1 or of a molding produced by a process as claimed in any of claims 2 to 5 for the epoxidation of an olefin, preferably for the preparation of propylene oxide starting from propylene and hydrogen peroxide.

8. The use of a mixture containing at least one alcohol and water as a pasting agent, in combination with a tetraalkoxysilane as a binder, for the preparation of a moldable mixture which contains a titanium silicalite.

## Revendications

1. Objet façonné contenant une silicalite de titane, pouvant être obtenu par un procédé comprenant les étapes suivantes:
(I) addition, á un mélange contenant une silicalite de titane et un tétraalcoxysilane ou un mélange d'au moins deux de ces dernières, d'un mélange contenant au moins un alcool et de l'eau, et
(II) malaxage, façonnage, séchage et calcination du mélange additionné selon l'étape (I),
dans lequel le mélange de l'étape (I) est en outre additionné d'un polymère hydrophile organique ou d'un mélange d'au moins deux d'entre eux.

2. Procédé de fabrication d'un objet façonné contenant au moins une silicalite de titane, qui comprend les étapes suivantes:
(I) addition, à un mélange contenant une silicalite de titane et un tétraalcoxysilane ou un mélange d'au moins deux de ces dernières, d'un mélange contenant au moins un alcool et de l'eau, et
(II) malaxage, façonnage, séchage et calcination du mélange additionné selon l'étape (I),
dans lequel le mélange de l'étape (I) est en outre additionné d'un polymère hydrophile organique ou d'un mélange d'au moins deux d'entre eux.

3. Procédé selon la revendication 2, dans lequel l'alcool du mélange contenant au moins un alcool et de l'eau conïcide avec l'alcool se trouvant dans l'ester d'acide métallique.

4. Procédé selon la revendication 2 ou 3, dans lequel le mélange obténu dans l'étape (I) est façonné par boudinage ou par extrusion.

5. Procédé selon l'une des revendications 2 à 4, dans lequel l'objet façonné contenant une silicalite de titane comprend des micropores, des mésopores, des macropores, des micropores et des mésopores, des micropores et des macropores, ou des micropores, des mésopores et des macropores.

6. Utilisation de l'objet façonné selon la revendication 1 ou d'un objet façonné fabriqué par un procédé selon l'une des revendication 2 à 5 ou d'un mélange d'au moins deux d'entre eux pour époxyder des composés organiques ayant au moins une double liaison C-C, pour hydroxyler des composés organiques aromatiques ou pour convertir des alcanes en alcools, cétones, aldéhydes et acides.

7. Utilisation de l'objet façonné selon la revendication 1 ou d'un objet façonné fabriqué par un procédé selon l'une des revendication 2 à 5 pour l'époxydation d'une olefine, de préférence pour fabriquer de l'oxyde de propylène à partir de propylène et de peroxyde d'hydrogène.

8. Utilisation d'un mélange contenant au moins un alcool et de l'eau en tant qu'agent de préparation de pâte, en combinaison avec un tétraalcoxysilane servant de liant, pour fabriquer un mélange pouvant être façonné et contentant une silicalite de titane.
